# EUROPÄISCHE PATENTSCHRIFT

(11) **EP 0 793 958 B1**
(45) Veröffentlichungstag und Bekanntmachung des Hinweises auf die Patenterteilung: **02.06.2004**
(21) Anmeldenummer: 97103306.3
(22) Anmeldetag: 28.02.1997
(51) Int. Cl.: A61K 31/215, A61K 9/16

(54) **Verfahren zur Herstellung Von Fenofibrat-Präparaten**
Process for preparing a composition containing fenofibrate
Procédé de préparation d'un composition contenant du fénofibrate

(30) Priorität: 06.03.1996 DE 19608750
(43) Veröffentlichungstag der Anmeldung: 10.09.1997
(73) Patentinhaber: MERCK PATENT GmbH, 64293 Darmstadt (DE)
(72) Erfinder: Keil, Mathias, Dr. rer. nat., 82538 Geretsried (DE); Bernhard, Georg, 87700 Memmingen (DE)

(56) Entgegenhaltungen:
- EP-A- 0 256 933
- US-A- 4 895 726
- SHEU, MING THAU ET AL.: "Characterization and dissolution of fenofibrate solid dispersion systems" INT. J. PHARM., Bd. 103, Nr. 2, 1994, Seiten 137-146, XP002091325

## Beschreibung

Die Erfindung betrifft ein Verfahren zur Herstellung von Fenofibrat-Präparaten, durch welches in einfacher Weise Fenofibrat-Präparate mit etwa gleich guter Bioverfügbarkeit und daraus folgend gleich guter Wirkung wie die der nach dem Stand der Technik hergestellten erhalten werden können.

Fenofibrat, mit der chemischen Bezeichnung 2-{4'-[4"--(Chlor)-benzoyl]-phenoxy}-2-{methyl}-propionsäure-isopropylester, ist ein bekannter Lipidsenker.

Aus der EP-PS 330 532 ist ein Verfahren zur Herstellung von Fenofibrat-Präparaten, bei welchem
(i) das Fenofibrat und ein festes, oberflächenaktives Mittel innig vermischt und hiernach einer gemeinsamen Strahlmühlenbehandlung unterzogen werden
(ii) zu dem erhaltenen Gemisch Lactose und Stärke zugegeben werden,
(iii) das Ganze in Gegenwart von Wasser granuliert und
(iv) bis zur Erzielung eines Granulates, das höchstens 1% Wasser enthält, getrocknet wird,
(v) das Granulat kalibriert wird und
(vi) Polyvinylpyrrolidon und Magnesiumstearat zugesetzt werden,
bekannt. Es wird behauptet, daß zur Verbesserung der Bioverfügbarkeit des Fenofibrates das gemeinsame Mahlen (Comikronisieren) von Fenofibrat und oberflächenaktivem Mittel unerläßlich sei (Seite 2, Zeilen 21 bis 22).

Ferner ist in der DE-PS 35 03 681 ein in Wasser quellbares, wasserunlösliches Polymer, welches mit einer biologisch aktiven Substanz oder einer Substanz, die in vivo in eine solche umgewandelt wird, beladen ist, erhältlich durch Herstellen und Mahlen einer Mischung dieser Substanz mit einem in Wasser quellbaren, wasserunlöslichen Polymer in einem Gewichtsverhältnis der genannten Substanz : Polymer von 1 : 0,1 bis 1 : 100, wobei das Polymer vernetztes Polyvinylpyrrolidon oder vernetzte Natriumcarboxymethylcellulose sein kann, beschrieben.

Weiterhin sind aus der DE-PS 31 52 519 Arzneimittel mit verzögerter Freisetzung zur oralen Verabreichung, enthaltend eine Wirkstoffschicht mit Bindemitteln und eine wasserdurchlässige poröse Ummantelung, deren neutraler Kern aus inerten Bindemitteln, ausgewählt aus der Gruppe Rohrzucker und Milchzukker, gegebenenfalls Stärke, besteht, und bei denen der neutrale Kern mit einer ersten wirkstoffhaltigen Schicht ummantelt ist, die Fenofibrat und/oder seine Derivate im Gemisch mit einem Bindemittel aus der Gruppe Talcum, Siliciumdioxyd oder deren Gemischen sowie Stearinsäure enthält, und die Granula eine zweite, äußere Schicht aufweisen, die aus einem mikroporösen Mantel besteht, gebildet unter anderen mit Polyvinylpyrrolidon, bekannt.

Außerdem ist in der EP-A1-256 933 ein Verfahren zur Herstellung eines Medikamentes in Körnchenform beschrieben, bei welchem in einer Stufe ein neutraler Kern mit einer äußeren feuchten klebenden Schicht, die unter anderem auf der Basis von Polyvinylpyrrolidon sein kann, angefeuchtet wird und dann in einer weiteren Stufe Fenofibrat-Mikroteilchen auf den angefeuchteten Kern, zweckmäßig durch Aufsprühen, aufgebracht werden und das Ganze getrocknet wird.

Nahezu dasselbe Verfahren betreffen die US-PS 4 800 079 und 4 961 890 sowie die FR-A1-2 602 423.

In der FR-A1-2 617 047 ist ein Fenofibrat-Präparat angegeben, welches Fenofibrat und ein oberflächenaktives Mittel sowie Dimethylisosorbid und gegebenenfalls ein Geliermittel und Excipienten in Kapseln enthält.

Ferner ist aus der GB-PS 931 147 ein Verfahren zur Herstellung einer Retardzubereitung durch Schmelzen von Polyvinylpyrrolidon, Fettsäuren und dergleichen und einem Wirkstoff, Tröpfchenbildung aus der Schmelze, Sprühen und Pelletbildung bekannt.

Weiterhin ist in der US-PS 4 925 672 eine Kombination von Verapamil und Fenofibrat, wobei der Fenofibrat-Teil auch Polyvinylpyrrolidon enthalten kann, beschrieben. Über das Verfahren ist nichts gesagt.

Außerdem sind in "Rote Liste" 1996, Präparat Nr. 58 029 (Normalip pro) Kapseln beschrieben, welche mikronisiertes Fenofibrat, quervernetztes Polyvinylpyrrolidon (Crospovidon) und Natriumdodecylsulfat enthalten. Das Herstellungsverfahren ist nicht angegeben, in Fachkreisen ist es jedoch bekannt, daß die Herstellung nach dem Verfahren der oben erörterten EP-PS 330 532 erfolgt.

Der Erfindung liegt die Aufgabe zugrunde, ein Verfahren zur Herstellung von Fenofibrat-Präparaten unter Verwendung von Fenofibrat, oberflächenaktiven Mitteln und Polyvinylpyrrolidon sowie gegebenenfalls weiteren Hilfsstoffen und unter Anwendung eines Mischens sowie Granulierens und anschließenden Trocknens, durch welches überraschenderweise einfacher als nach dem Stand der Technik Fenofibrat-Präparate mit etwa gleich guter therapeutischer Wirkung wie die der nach dem Stand der Technik erhaltenen erzielt werden können, zu schaffen.

Das Obige wurde überraschenderweise ohne gemeinsames Mahlen von Fenofibrat und festen oberflächenaktiven Mitteln erfindungsgemäß erreicht.

Gegenstand der Erfindung ist ein Verfahren zur Herstellung von Fenofibrat-Präparaten unter Verwendung von Fenofibrat, oberflächenaktiven Mitteln und Polyvinylpyrrolidon sowie gegebenenfalls 1 oder mehr weiteren Hilfsstoff(en) und unter Anwendung eines Mischens sowie Granulierens und anschließenden Trocknens, welches dadurch gekennzeichnet ist, daß zunächst Fenofibratteilchen mit Polyvinylpyrrolidon- und quervernetzten Polyvinylpyrrolidonteilchen sowie gegebenenfalls weiteren Hilfsstoffteilchen vermischt werden und dann die erhaltene Mischung mit einer wäßrigen Lösung von 1 oder mehr oberflächenaktiven Mittel(n) in einem Mengenanteil von mindestens 1,5 Gew.-%, bezogen auf das herzustellende trockene Granulat, granuliert und das Granulat getrocknet wird.

Vorzugsweise wird als Fenofibrat mikronisiertes verwendet.

Gegenüber der Lehre von EP-PS 330 532, zur Erzielung einer optimalen Bioverfügbarkeit ein gemeinsames Mahlen von Fenofibrat und einem festen oberflächenaktiven Mittel durchführen zu müssen, ist es überraschend, daß dies erfindungsgemäß auch ohne dieses dadurch gelingt, daß das getrennt gemahlene Fenofibrat mit Polyvinylpyrrolidon und quervernetztem Polyvinylpyrrolidon ohne Mahlen lediglich vermischt wird und diese Mischung mit dem in wäßriger Lösung eingesetzten oberflächenaktiven Mittel granuliert wird und nicht etwa das Granulieren erst nach dem Einbringen des oberflächenaktiven Mittels erfolgt.

Das in Mischung mit dem Fenofibrat vorliegende Polyvinylpyrrolidon ermöglicht erst den Aufbau einer Granulatstruktur beim Besprühen mit der Lösung des/der oberflächenaktiven Mittel[s]. Es ist anzunehmen, daß es auch eine Hydrophilisierung des Fenofibrates bewirkt, was zu einer besseren Resorption und damit Erhöhung der Bioverfügbarkeit führt. Die Mindestmenge des der oberflächenaktiven Mittel[s] ist kritisch, da unterhalb 1,5 Gew.-% kein befriedigendes therapeutisches Ergebnis zu erzielen war. Die verfahrenstechnische Vereinfachung beim erfindungsgemäßen Verfahren gegenüber dem der EP-PS 330 532 liegt hauptsächlich darin, daß erfindungsgemäß alle Hilfsstoffe in einem in ein und derselben Stufe mit dem Fenofibrat vermischt werden können, während beim genannten bekannten Verfahren wegen des zwingend vorgeschriebenen gemeinsamen Mahlens (Co-mikronisierens) des Fenofibrates und des festen oberflächenaktiven Mittels die weiteren Hilfsstoffe zwingend in einer zusätzlichen getrennten Stufe zugemischt werden müssen. Außerdem bedingt beim erfindungsgemäßen Verfahren das Mikronisieren allein des Fenofibrates eine Verringerung des Mikronisiervolumens gegenüber dem gemeinsamen Mikronisieren von Fenofibrat und festem oberflächenaktiven Mittel und damit einen geringeren Energieaufwand.

Auch von der DE-PS 35 03 681 hebt sich das erfindungsgemäße Verfahren grundlegend dadurch ab, daß im Gegensatz zum dort beschriebenen gemeinsamen Mahlen von Wirkstoff und Hilfsstoffen, wie quervernetztem Polyvinylpyrrolidon (Patentanspruch 1, Seite 2, Zeilen 23 bis 33 und Seite 3, Zeilen 34 bis 37), mit der Behauptung, daß die Verminderung der Teilchengröße von Arzneimitteln bei deren getrenntem Mahlen häufig nicht wirksam genug sei (Seite 2, Zeilen 7 bis 10), wie bereits gesagt, ein getrenntes Mahlen des Fenofibrates erfolgt und des gemahlene Fenofibrat mit den Hilfsstoffen nur physikalisch vermischt wird. Ferner ist im Gegensatz zur DE-PS 35 03 681 mit der Verwendung nur eines wasserunlöslichen Polymers beim erfindungsgemäßen Verfahren die Verwendung des wasserlöslichen Polyvinylpyrrolidones zum Mischen mit dem Fenofibrat unerläßlich, um die gestellte Aufgabe zu lösen. Auch wird beim erfindungsgemäßen Verfahren das Granulieren mit einem oberflächenaktiven Mittel durchgeführt, welches in der genannten Druckschrift überhaupt nicht verwendet wird.

Dabei sind auch die Produktcharakteristika verschieden, indem beim Verfahren der DE-PS 35 03 681 und der EP-PS 330 532 beim gemeinsamen Mahlen von Wirkstoff und Hilfsstoffen eine Amorphisierung des Wirkstoffes stattfindet, während beim bloßen Mischen beim erfindungsgemäßen Verfahren das Fenofibrat kristallin bleibt, wie es eigene Versuche mit einem Vergleich von unvermahlenem Fenofibrat und allein gemahlenem Fenofibrat ergaben.

Im Gegensatz zur DE-PS 31 52 519, nach welcher sich das Fenofibrat und das Polyvinylpyrrolidon an verschiedenen Stellen befinden, nämlich das erstere in der ersten Schicht und das letztere in der äußeren Schicht, wobei von einem Vermischtsein der beiden keine Rede ist, erfolgt beim erfindungsgemäßen Verfahren ein Vermischen der Fenofibratteilchen mit Polyvinylpyrrolidonteilchen und darüberhinaus quervernetzten Polyvinylpyrrolidonteilchen. Von der Verwendung von quervernetztem Polyvinylpyrrolidon und einem oberflächenaktiven Mittel ist in der genannten Druckschrift keine Rede. Nach deren Verfahren könnte quervernetztes Polyvinylpyrrolidon gar nicht verwendet werden, weil ausweislich Seite 3, Zeilen 5 bis 51 die Polymere in Lösung aufgebracht werden.

Im Gegensatz zur EP-A1-256 933, nach welcher ein Aufbringen, wie Aufsprühen, von Fenofibrat auf das lediglich als Bindemittel wirkende Polyvinylpyrrolidon durchgeführt wird, er folgt beim erfindungsgemäßen Verfahren ein Vermischen des Fenofibrates mit dem Polyvinylpyrrolidon und darüberhinaus mit quervernetztem Polyvinylpyrrolidon. Dabei besteht auch der Unterschied zu EP-A1-256 933, in welcher kein quervernetztes Polyvinylpyrrolidon verwendet wird, - was nach deren Verfahren sogar ausgeschlossen ist, weil ein in Wasser lösliches Bindemittel verwendet werden muß -, daß beim erfindungsgemäßen Verfahren zwingend quervernetzte Polyvinylpyrrolidonteilchen mit zugemischt werden müssen. Ein noch weiterer Unterschied liegt darin, daß nach dem erfindungsgemäßen Verfahren im Gegensatz zu dem der genannten Druckschrift in einer weiteren Stufe mit einem oberflächenaktiven Mittel granuliert wird.

Das erfindungsgemäße Verfahren unterscheidet sich von dem der FR-A1-2 617 047 grundlegend darin, daß im ersteren das oberflächenaktive Mittel durch Granulieren eingebracht wird und dies auch nur, nachdem die Fenofibratteilchen mit den Polyvinylpyrrolidon- und quervernetzten Polyvinylpyrrolidonteilchen vermischt sind. Dabei besteht der weitere Unterschied, daß die beiden letztgenannten Substanzen in der genannten Druckschrift ganz fehlen.

Von der US-PS 4 925 672, in welcher überhaupt kein Verfahren beschrieben ist, unterscheidet sich die Erfindung grundlegend darin, daß es sich um eine Verfahrenserfindung handelt, wozu noch hinzukommt, daß im Gegensatz zur genannten Druckschrift, in welcher von einem Gehalt an quervernetztem Polyvinylpyrrolidon und einem oberflächenaktiven Mittel keine Rede ist, auch diese Substanzen mit verwendet werden.

Vorzugsweise wird/werden als oberflächenaktive[s] Mittel [ein] anionische[s] verwendet. Dabei ist es bevorzugt, als anionische[s] oberflächenaktive[s] Mittel [ein] Alkalialkylsulfat(e), insbesondere Natriumlaurylsulfat, zu verwenden.

Es ist auch bevorzugt, als Polyvinylpyrrolidon ein solches mit einem K-Wert von 10 bis 96, insbesondere 25 bis 35, zu verwenden.

Ferner ist es bevorzugt, als quervernetztes Polyvinylpyrrolidon ein solches mit einer spezifischen Oberfläche (BET) von 0,1 bis 1,5 m²/g, insbesondere 0,5 bis 1,5 m²/g, ganz besonders 0,7 bis 1,1 m²/g, zu verwenden.

Vorzugsweise wird als Fenofibrat ein solches mit Teilchengrößen von 100% ≦ 20 µm verwendet.

Es ist auch bevorzugt, als Polyvinylpyrrolidon ein solches mit Teilchengrößen von 100% ≦ 500 µm zu verwenden.

Ferner ist es bevorzugt, als quervernetztes Polyvinylpyrrolidon ein solches mit Teilchengrößen von 100% ≦ 500 µm zu verwenden.

Vorzugsweise wird das Fenofibrat in Mengenanteilen von 65 bis 85 Gew.-%, insbesondere 70 bis 80 Gew.-%, bezogen auf das trockene Granulat, verwendet.

Ferner ist es bevorzugt, das/die oberflächenaktive(n) Mittel in Mengenanteilen von 1,5 bis 7 Gew.-%, insbesondere 2 bis 5 Gew.-%, bezogen auf das trockene Granulat, zu verwenden.

Es ist auch bevorzugt, das/die oberflächenaktive(n) Mittel in 1 bis 5 gew.-%-iger, insbesondere 2 bis 3 gew.-%-iger, Konzentration einzusetzen.

Weiterhin ist es bevorzugt, das Polyvinylpyrrolidon in Mengenanteilen von 2 bis 6 Gew.-%, insbesondere 3 bis 5 Gew.-%, bezogen auf das trockene Granulat, zu verwenden.

Es ist auch bevorzugt, das quervernetzte Polyvinylpyrrolidon in Mengenanteilen von 10 bis 30 Gew.-%, insbesondere 15 bis 25 Gew.-%, bezogen auf das trockene Granulat, zu verwenden.

Der/die gegebenenfalls verwendete[n] weitere[n] Hilfsstoff(e) kann/können [ein] in der pharmazeutischen Technik übliche[r] sein. Beispiele sind Stärke, mikrokristalline Cellulose, Lactose und Magnesiumstearat. Das Zumischen dieses/dieser weiteren Hilfsstoffe[s] zum Fenofibrat wird zweckmäßig zusammen mit dem Polyvinylpyrrolidon und quervernetzten Polyvinylpyrrolidon vorgenommen, es kann aber auch in einer späteren oder früheren Phase erfolgen, in welchen Fällen aber die Verfahrensvereinfachung geringer ist.

Nach einer Ausführungsform des erfindungsgemäßen Verfahrens wird das erhaltene Granulat in Kapseln, insbesondere Hartgelatinekapseln, gefüllt.

Das Trocknen des Granulates, vorteilhaft bis zu einem Restfeuchtegehalt von höchstens 2,5 Gew.-%, insbesondere höchstens 2,0 Gew.-%, und sein gegebenenfalls erfolgendes Einfüllen in Kapseln kann/können in an sich bekannter Weise durchgeführt werden.

Die so hergestellten Granulate beziehungsweise Kapseln mit ihrer gleich guten Wirkung wie die der nach dem Stand der Technik hergestellten können mit Erfolg therapeutisch, insbesondere als Lipidsenker, verwendet werden.

Die Erfindung wird anhand des folgenden Beispieles näher erläutert.

### Beispiel

Es wurde eine Mischung aus 90 kg mikronisiertem Fenofibrat mit Teilchengrößen von 100% ≦ 15 µm und dabei 97 bis 95% ≦ 5 µm, 4,5 kg Polyvinylpyrrolidonteilchen DAB 10 mit einem K-Wert von 27 bis 32 und mit Teilchengrößen von 95% ≦ 250 µm und dabei 10% < 50 µm und 22,5 kg quervernetzten Polyvinylpyrrolidonteilchen DAB 10 mit einer spezifischen Oberfläche (BET) von 0,9 m²/g und mit Teilchengrößen von mindestens 98% ≦ 250 µm und höchstens 60% ≦ 50 µm durch ein 0,8 mm Sieb gedrückt und 10 Minuten lang gemischt. Getrennt davon wurde eine Lösung aus 2,475 kg Natriumlaurylsulfat NF 18 und 101,25 kg gereinigtem Wasser bereitet. Die erstgenannte Pulvermischung wurde dann mit der letztgenannten Lösung in einem Wirbelschichtgranulator mit einer Einlaßtemperatur von etwa 20 bis 40°C (Auslaßtemperatur: 20 ± 5°C) granuliert. Das feuchte Granulat wurde in einem Trockner bei einer Einlaßtemperatur von etwa 50 ± 5°C zu einem Restfeuchtegehalt von etwa 1,5 ± 0,5% getrocknet. Das getrocknete Granulat wurde durch ein 0,5 mm Sieb gedrückt. Dann erfolgte noch ein 10 Minuten langes Nachmischen.

Das so erhaltene Granulat wurde in einer Kapselfüllmaschine in opake HS-Hartgelatinekapseln No. 1 mit einer türkisblauen Kappe und einem weißen Körper eingefüllt. Mit dem Granulat mit einem Gesamtgewicht von 119,475 kg wurden 450.000 Kapseln mit einem Kapselinhalt von je 265 mg gefüllt.

Die Bestimmung der wissenschaftlich anerkannten therapeutischen Zielgrößen Cₘₐₓ , tₘₐₓ , AUCₜ und AUC ergab keine signifikante Abweichung zwischen dem vorstehend erfindungsgemäß hergestellten Fenofibrat-Präparat und dem nach Beispiel 1 der EP-PS 330 532 hergestellten.

## Patentansprüche

1. Verfahren zur Herstellung von Fenofibrat-Präparaten unter Verwendung von Fenofibrat, oberflächenaktiven Mitteln und Polyvinylpyrrolidon sowie gegebenenfalls 1 oder mehr weiteren Hilfsstoff(en) und unter Anwendung eines Mischens sowie Granulierens und anschließenden Trocknens, **dadurch gekennzeichnet, daß** man zunächst Fenofibratteilchen mit Polyvinylpyrrolidon- und quervernetzten Polyvinylpyrrolidonteilchen sowie gegebenenfalls weiteren Hilfsstoffteilchen vermischt und dann die erhaltene Mischung mit einer wäßrigen Lösung von 1 oder mehr oberflächenaktiven Mittel(n) in einem Mengenanteil von mindestens 1,5 Gew.-%, bezogen auf das herzustellende trockene Granulat, granuliert und das Granulat trocknet.

2. Verfahren nach Anspruch 1, **dadurch gekennzeichnet, daß** man als Fenofibrat mikronisiertes verwendet.

3. Verfahren nach Anspruch 1 oder 2, **dadurch gekennzeichnet, daß** man als oberflächenaktive[s] Mittel [ein] anionische[s] verwendet.

4. Verfahren nach Anspruch 3, **dadurch gekennzeichnet, daß** man als anionische[s] oberflächenaktive[s] Mittel [ein] Alkalialkylsulfat(e), insbesondere Natriumlaurylsulfat, verwendet.

5. Verfahren nach Anspruch 1 bis 4, **dadurch gekennzeichnet, daß** man als Polyvinylpyrrolidon ein solches mit einem K-Wert von 10 bis 96, insbesondere 25 bis 35, verwendet.

6. Verfahren nach Anspruch 1 bis 5, **dadurch gekennzeichnet, daß** man als quervernetztes Polyvinylpyrrolidon ein solches mit einer spezifischen Oberfläche (BET) von 0,1 bis 1,5 m²/g, insbesondere 0,5 bis 1,5 m²/g, verwendet.

7. Verfahren nach Anspruch 1 bis 6, **dadurch gekennzeichnet, daß** man als Fenofibrat ein solches mit Teilchengrößen von 100% ≦ 20 µm verwendet.

8. Verfahren nach Anspruch 1 bis 7, **dadurch gekennzeichnet, daß** man als Polyvinylpyrrolidon ein solches mit Teilchengrößen von 100% ≦ 500 µm verwendet.

9. Verfahren nach Anspruch 1 bis 8, **dadurch gekennzeichnet, daß** man als quervernetztes Polyvinylpyrrolidon ein solches mit Teilchengrößen von 100% ≦ 500 µm verwendet.

10. Verfahren nach Anspruch 1 bis 9, **dadurch gekennzeichnet, daß** man das Fenofibrat in Mengenanteilen von 65 bis 85 Gew.-%, insbesondere 70 bis 80 Gew.-%, bezogen auf das trockene Granulat, verwendet.

11. Verfahren nach Anspruch 1 bis 10, **dadurch gekennzeichnet, daß** man das/die oberflächenaktive(n) Mittel in Mengenanteilen von 1,5 bis 7 Gew.-%, insbesondere 2 bis 5 Gew.-%, bezogen auf das trockene Granulat, verwendet.

12. Verfahren nach Anspruch 1 bis 11, **dadurch gekennzeichnet, daß** man das/die oberflächenaktive(n) Mittel in 1 bis 5 gew.-%-iger, insbesondere 2 bis 3 gew.-%--iger, Konzentration einsetzt.

13. Verfahren nach Anspruch 1 bis 12, **dadurch gekennzeichnet, daß** man das Polyvinylpyrrolidon in Mengenanteilen von 2 bis 6 Gew.-%, insbesondere 3 bis 5 Gew.-%, bezogen auf das trockene Granulat, verwendet.

14. Verfahren nach Anspruch 1 bis 13, **dadurch gekennzeichnet, daß** man das quervernetzte Polyvinylpyrrolidon in Mengenanteilen von 10 bis 30 Gew.-%, insbesondere 15 bis 25 Gew.-%, bezogen auf das trockene Granulat, verwendet.

15. Verfahren nach Anspruch 1 bis 14, **dadurch gekennzeichnet, daß** man das erhaltene Granulat in Kapseln, insbesondere Hartgelatinekapseln füllt.

## Claims

1. Process for the preparation of fenofibrate preparations using fenofibrate, surface-active agents and polyvinylpyrrolidone and optionally 1 or more further auxiliary or auxiliaries and using mixing and granulation and subsequent drying, **characterised in that** firstly fenofibrate particles are mixed with polyvinylpyrrolidone particles and crosslinked polyvinylpyrrolidone particles and optionally further auxiliary particles, and the resultant mixture is then granulated with an aqueous solution of 1 or more surface-active agent(s) in a proportion of at least 1.5% by weight, based on the dry granules to be produced, and the granules are dried.

2. Process according to Claim 1, **characterised in that** the fenofibrate used is micronised fenofibrate.

3. Process according to Claim 1 or 2, **characterised in that** the surface-active agent(s) used is (are) [an] anionic surface-active agent(s).

4. Process according to Claim 3, **characterised in that** the anionic surface-active agent(s) used is (are) [an] alkali metal alkylsulfate(s), in particular sodium laurylsulfate.

5. Process according to Claims 1 to 4, **characterised in that** the polyvinylpyrrolidone used is one having a K value of from 10 to 96, in particular from 25 to 35.

6. Process according to Claims 1 to 5, **characterised in that** the crosslinked polyvinylpyrrolidone used is one having a specific surface area (BET) of from 0.1 to 1.5 m²/g, in particular from 0.5 to 1.5 m²/g.

7. Process according to Claims 1 to 6, **characterised in that** the fenofibrate used is one having particle sizes of 100% ≤ 20 µm.

8. Process according to Claims 1 to 7, **characterised in that** the polyvinylpyrrolidone used is one having particle sizes of 100% ≤ 500 µm.

9. Process according to Claims 1 to 8, **characterised in that** the crosslinked polyvinylpyrrolidone used is one having particle sizes of 100% ≤ 500 µm.

10. Process according to Claims 1 to 9, **characterised in that** the fenofibrate is used in proportions of from 65 to 85% by weight, in particular from 70 to 80% by weight, based on the dry granules.

11. Process according to Claims 1 to 10, **characterised in that** the surface-active agent(s) is (are) used in proportions of from 1.5 to 7% by weight, in particular from 2 to 5% by weight, based on the dry granules.

12. Process according to Claims 1 to 11, **characterised in that** the surface-active agent(s) is (are) employed in a concentration of from 1 to 5% by weight, in particular from 2 to 3% by weight.

13. Process according to Claims 1 to 12, **characterised in that** the polyvinylpyrrolidone is used in proportions of from 2 to 6% by weight, in particular from 3 to 5% by weight, based on the dry granules.

14. Process according to Claims 1 to 13, **characterised in that** the crosslinked polyvinylpyrrolidone is used in proportions of from 10 to 30% by weight, in particular from 15 to 25% by weight, based on the dry granules.

15. Process according to Claims 1 to 14, **characterised in that** the granules obtained are filled into capsules, in particular hard gelatine capsules.

## Revendications

1. Procédé pour la préparation de préparations en utilisant du fénofibrate, des agents tensioactifs et du polyvinylpyrrolidone et optionnellement 1 ou plusieurs autre auxiliaire ou autres auxiliaires et en utilisant un mélange et une granulation et ensuite un séchage, **caractérisé en ce que** tout d'abord des particules de fénofibrate sont mélangées avec des particules de polyvinylpyrrolidone et des particules de polyvinylpyrrolidone réticulées et optionnellement d'autres particules auxiliaires, et le mélange résultant est ensuite granulé avec une solution aqueuse de 1 ou plusieurs agent(s) tensioactif(s) dans un proportion d'au moins 1,5% en poids, sur la base des granules sèches à produire, et les granules sont séchées.

2. Procédé selon la revendication 1, **caractérisé en ce que** le fénofibrate utilisé est un fénofibrate micronisé.

3. Procédé selon la revendication 1 ou 2, **caractérisé en ce que** le ou les agent(s) tensioactif(s) utilisé(s) est/sont un ou des agent(s) tensioactif(s) anioniques.

4. Procédé selon la revendication 3, **caractérisé en ce que** le ou les agent(s) tensioactif(s) utilisé(s) est/sont un ou des alkylsufate(s) de métal alcalin, en particulier laurylsulfate de sodium.

5. Procédé selon les revendications 1 à 4, **caractérisé en ce que** le polyvinylpyrrolidone utilisé est un composé ayant une valeur K de 10 à 98, en particulier de 25 à 35.

6. Procédé selon les revendications 1 à 5, **caractérisé en ce que** le polyvinylpyrrolidone réticulé utilisé est un composé ayant une aire de surface spécifique (BET) de 0,1 à 1,5 m²/g, en particulier de 0,5 à 1,5 m²/g.

7. Procédé selon les revendications 1 à 6, **caractérisé en ce que** le fénofibrate utilisé est un composé ayant des dimensions de particules de 100% ≤ 20 µm.

8. Procédé selon les revendications 1 à 7, **caractérisé en ce que** le polyvinylpyrrolidone utilisé est un composé ayant des dimensions de particules de 100% ≤ 500 µm.

9. Procédé selon les revendications 1 à 8, **caractérisé en ce que** le polyvinylpyrrolidone réticulé utilisé est un composé ayant des dimensions de particules de 100% ≤ 500 µm.

10. Procédé selon les revendications 1 à 9, **caractérisé en ce que** le fénofibrate est utilisé dans des proportions de 65 to 85% en poids, en particulier de 70 to 80% en poids, sur la base des granules sèches.

11. Procédé selon les revendications 1 à 10, **caractérisé en ce que** le ou les agent(s) tensioactif(s) est/sont employé(s) selon une concentration de 1.5 to 7% en poids, en particulier de 2 to 5% en poids, sur la base des granules sèches.

12. Procédé selon les revendications 1 à 11, **caractérisé en ce que** le ou les agent(s) tensioactif(s) est/sont employé(s) selon une concentration de 1 to 5% en poids, en particulier de 2 to 3% en poids.

13. Procédé selon les revendications 1 à 12, **caractérisé en ce que** le polyvinylpyrrolidone est utilisé dans des proportions de 2 to 6% en poids, en particulier de 3 to 5% en poids, sur la base des granules sèches.

14. Procédé selon les revendications 1 à 13, **caractérisé en ce que** le polyvinylpyrrolidone réticulé est utilisé dans des proportions de 10 to 30% en poids, en particulier de 15 to 25% en poids, sur la base des granules sèches.

15. Procédé selon les revendications 1 à 14, **caractérisé en ce que** les granules obtenues remplissent des capsules, en particulier des capsules de gélatine dure.
